# EUROPEAN PATENT APPLICATION

(11) **EP 4 834 954 A1**
(43) Date of publication of application: **04.11.2026**
(21) Application number: 24909829.4
(22) Date of filing: 14.08.2024
(51) Int. Cl.: C08F 10/00, C08F 110/06, C08F 2/34

(54) **INTERNAL ELECTRON DONOR, SOLID CATALYST COMPONENT, POLYMERIZATION CATALYST, AND USE THEREOF**

(30) Priority: 27.12.2023 CN 202311822655
(71) Applicant: China Energy Investment Corporation Limited, Beijing 100011 (CN); National Institute of Clean-and-Low-Carbon Energy, Beijing 102211 (CN)
(72) Inventor: HUANG, Jinhua, Beijing 102211 (CN); YU, Peiqian, Beijing 102211 (CN); YU, Yue, Beijing 102211 (CN); FENG, Liuhai, Beijing 102211 (CN); SHI, Mengxue, Beijing 102211 (CN); LI, Yulian, Beijing 102211 (CN); BIAN, Yufei, Beijing 102211 (CN); ZHENG, Zhigang, Beijing 102211 (CN); CHEN, Linfeng, Beijing 102211 (CN)
(74) Representative: Bergenstråhle & Partners AB
(86) International application number: PCT/CN2024/111968
(87) International publication number: WO 2025/138878

(57) **Abstract**

The present disclosure belongs to the technical field of olefin polymerization catalysts, and particularly relates to an internal electron donor, a solid catalyst component, a polymerization catalyst and use thereof in olefin polymerization. The internal electron donor is at least one selected from the compounds represented by Formula (I), wherein R₁ to R₁₆ are the same or different, and each is independently selected from H, halogen, C₁-C₁₀ alkyl, cycloalkyl, alkenyl, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; two or more of R₁ to R₁₄ may be linked together to form cyclic structure(s) or unsaturated bond(s). The polymerization catalyst prepared from the internal electron donor provided in the present disclosure exhibits high catalytic activity and good hydrogen response, and resultant polymer has high isotactic indexes, low xylene-soluble fraction and broad molecular weight distribution.

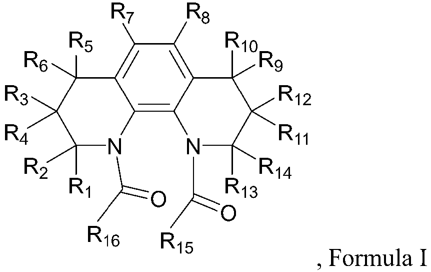

## Description

### TECHNICAL FIELD

The present disclosure relates to the technical field of olefin polymerization catalysts, and particularly to an internal electron donor, a solid catalyst component, a polymerization catalyst and use thereof in olefin polymerization.

### BACKGROUND

It is well known that the Ziegler-Natta catalysts used for olefin polymerization consist of three components: a magnesium chloride support, an internal electron donor compound, and a titanium compound. The internal electron donor compound can not only enhance the catalytic activity of the olefin polymerization catalyst, but also improve the stereoselectivity of the catalyst. Catalysts without internal electron donors exhibit significantly lower catalytic activity, and the resulting polymers fails to meet the requirements of practical application due to low isotactic index.

A variety of compounds have been widely used as internal electron donors for the preparation of Ziegler-Natta catalysts. Exemplary donor compounds include aromatic monoesters or diesters, such as diisobutyl phthalate or ethyl benzoate, etc., disclosed in US 4,784,983 A; diol esters disclosed in CN 1453298 A; diethers disclosed in EP 361,494 A; aromatic 1,2-phenylene diesters disclosed in US 61,141,902 A; and amides or amide esters disclosed in CN 108570120 A, etc.

In industrial production, these internal electron donor compounds exhibit certain deficiencies in practical applications. Catalysts adopting aromatic diester-based internal electron donors exhibit relatively low catalytic activity. Diether-based internal electron donors can endow catalysts with high catalytic activity and good hydrogen response, whereas the resultant polymers exhibit a narrow molecular weight distribution. Catalysts using aromatic 1,2-phenylene diester-based internal electron donors exhibit both good catalytic activity and hydrogen response, but yield polymers with a high xylene-soluble fraction. Catalysts employing amide or amide ester compounds as internal electron donors exhibit low catalytic activity and a high xylene-soluble fraction.

At present, conventional internal electron donor compounds for this application present various shortcomings in practical use. Accordingly, research on improved internal electron donor compounds has long been a major focus in this field. Under such circumstances, developing novel internal electron donors for olefin polymerization catalysts to address the aforesaid deficiencies of the prior art is of great practical significance..

### SUMMARY OF THE INVENTION

In order to solve the above-mentioned technical problems, the purpose of the present disclosure is to provide an internal electron donor, a solid catalyst component, a polymerization catalyst, and use thereof in olefin polymerization. A polymerization catalyst prepared using the novel internal electron donor exhibits the advantages of high catalytic activity and good hydrogen response, and a polymer obtained by using the catalyst in an olefin polymerization has the characteristics of high isotactic indexes, relatively low xylene-soluble fraction, and broad molecular weight distribution.

In order to achieve the above objects, the present disclosure provides the following technical solutions.

According to a first aspect, the present disclosure provides an internal electron donor for an olefin polymerization catalyst, wherein the internal electron donor is at least one selected from the compounds represented by Formula I below: 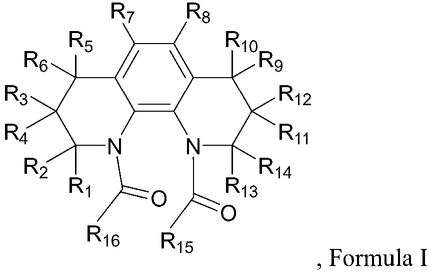 in Formula I:
R₁ to R₁₄ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl; any two or more of R₁ to R₁₄ may be linked together to form cyclic structure(s) or unsaturated bond(s);
R₁₅ and R₁₆ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl;
any hydrogen atom on the benzene ring of the above-mentioned aryl, arylalkyl or alkylaryl groups may each be optionally substituted with halogen.

In some embodiments, in Formula I, each of R₁₅ and R₁₆ is independently selected from phenyl, butyl and ethoxy.

In some embodiments, the internal electron donor is at least one selected from the compounds represented by Formula (II) below: 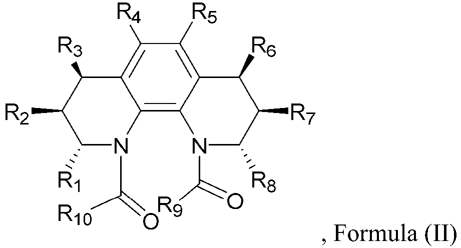 in Formula (II):
R₁ to R₈ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl; two or more of R₁ to R₈ may be linked together to form cyclic structure(s) or unsaturated bond(s);
R₉ and R₁₀ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl.

In some embodiments, in Formula (II), each of R₉ and R₁₀ is independently selected from phenyl, butyl and ethoxy.

According to a second aspect, the present disclosure provides a solid catalyst component for olefin polymerization, comprising: a titanium compound, a magnesium compound, and at least one of the above-mentioned internal electron donors.

In some embodiments, a method for preparing the solid catalyst component comprises: bringing a magnesium compound precursor, a titanium compound and at least one of the internal electron donors into contact and reaction to obtain the solid catalyst component; wherein,
the magnesium compound precursor is at least one selected from the compounds represented by Formula XₙMg(OR)₂₋ₙ, the compounds represented by Formula MgCl₂·mROH, MgCl₂/SiO₂ mixtures, MgCl₂/Al₂O₃ mixtures, and mixtures of magnesium halides and titanium alkoxides, wherein m ranges from 0.1 to 6 (e.g., 0.2, 0.5, 0.8, 1, 2, 3, 4 or 5), 0≦n≦2 (e.g., n is 0, 1 or 2), X is selected from halogen (e.g., fluorine, chlorine or bromine), R is selected from hydrogen and C₁-C₈ hydrocarbyl group (e.g., methyl, ethyl, propyl, n-butyl, isobutyl, 1-pentyl, 1-hexyl or 1-heptyl);
the titanium compound is represented by Formula TiXₙ(OR)₄₋ₙ, wherein R is selected from C₁-C₂₀ hydrocarbyl, X is selected from halogen, and n ranges from 1 to 4 (e.g., 1, 2, 3 or 4).

In some embodiments, non-limiting examples of the titanium compound include titanium tetrachloride, titanium tetrabromide, titanium tetraiodide and alkyl titanium halides. Exemplary alkyl titanium halides are selected from methoxy titanium trichloride, ethoxy titanium trichloride, propoxy titanium trichloride, n-butoxy titanium trichloride, dimethoxy titanium dichloride, diethoxy titanium dichloride, dipropoxy titanium dichloride, di-n-butoxy titanium dichloride, trimethoxy titanium chloride, triethoxy titanium chloride, tripropoxy titanium chloride, or tri-n-butoxy titanium chloride. These alkyl titanium halides may be used alone or in combination.

In the present disclosure, the magnesium compounds are well-known in the prior art, and will not be elaborated in detail. Exemplary magnesium compound can be derived from a magnesium compound precursor.

In the present disclosure, the method and specific process for preparing the solid catalyst component are conventional in the art, and will not be described in detail.

The content or amount of the internal electron donor in the solid catalyst component may be selected within conventional ranges known in the art.

According to a third aspect, the present disclosure provides an olefin polymerization catalyst, wherein the olefin is CH₂=CHR, in which R is selected from hydrogen and hydrocarbyl group having 1 to 12 carbon atoms, the catalyst comprises a product obtained by reacting the following starting materials:
(a) at least one of the above-mentioned solid catalyst components;
(b) at least one of the organo-aluminum compounds represented by Formula AlRₙX₍₃₋ₙ₎, wherein R is selected from hydrogen and a C₁-C₂₀ hydrocarbyl group; X is selected from halogen, and n is an integer satisfying 0≦n≦3;
(c) at least one of the alkoxysilane compounds represented by Formula RₙSi(OR¹)₄₋ₙ, wherein R and R¹ are the same or different, and each is independently selected from C₁-C₁₈ hydrocarbyl group and halogenated hydrocarbyl group, and C₁-C₁₀ substituent optionally containing heteroatom(s) selected from N, O, S, and P; and n is an integer satisfying 0≦n≦3.

In some embodiments, for the organo-aluminum compounds represented by Formula AlRₙX₍₃₋ₙ₎, each R is hydrogen or a C₁-C₁₂ hydrocarbyl group.

In some embodiments, the organo-aluminum compound may be an alkyl aluminum compound selected from trialkylaluminum compounds, preferably one or more selected from trimethylaluminum, triethylaluminum, triisobutylaluminum, tri-n-butylaluminum, tri-n-hexylaluminum and trioctylaluminum.

The amount of the organo-aluminum compound as a co-catalyst employed in the olefin polymerization catalysts is within conventional ranges well known in the art. For example, the molar ratio of the solid catalyst component to the organo-aluminum compound, Ti/Al, ranges from 1:5 to 1:400.

In some embodiments, for the alkoxysilane compound represented by Formula RₙSi(OR¹)₄₋ₙ, each of R and R¹ is independently selected from C₁-C₁₈ alkyl, C₃-C₁₈ cycloalkyl, C₆-C₁₈ aryl, and C₁-C₁₈ haloalkyl.

The alkoxysilane compound specifically include, but is not limited to tetramethoxysilane, tetraethoxysilane (TEOS), dimethyldimethoxysilane, dimethyldiethoxysilane, methyl tert-butyldimethoxysilane, methyl isopropyldimethoxysilane, diphenyloxydimethoxysilane, vinyltrimethoxysilane, methylcyclohexyldimethoxysilane, ethylcyclohexyldimethoxysilane, di-n-propyldimethoxysilane, diisopropyldimethoxysilane, di-n-butyldimethoxysilane, diisobutyldimethoxysilane, di-tert-butyldimethoxysilane, dicyclopentyldimethoxysilane (D donor), cyclopentyltrimethoxysilane, isopropyltrimethoxysilane, n-propyltrimethoxysilane, n-propyltriethoxysilane, ethyltriethoxysilane, diethylaminotriethoxysilane, cyclohexylpyrrolidinyldimethoxysilane, bis(pyrrolidinyl)-dimethoxysilane, bis(perhydroisoquinolinyl)dimethoxysilane, 2-ethylpiperidinyl-2-tert-butyldimethoxysilane, (1,1,1-trifluoro-2-propyl)(2-ethylpiperidinyl)dimethoxysilane, and (1,1,1-trifluoro-2-propyl)-(methyl)dimethoxysilane. The alkoxysilane compound is preferably one or more selected from dicyclopentyldimethoxysilane (D donor), methylcyclohexyldimethoxysilane, diisopropyldimethoxysilane, diisobutyldimethoxysilane, n-propyltrimethoxysilane, n-propyltriethoxysilane, tetraethoxysilane (TEOS), and diethylaminotriethoxysilane.

The amount of the alkoxysilane compound as an external electron donor component employed in the olefin polymerization catalysts is within conventional ranges well known in the art, and thus detailed descriptions are omitted herein.

According to a fourth aspect, the present disclosure provides use of the above-mentioned polymerization catalyst in olefin polymerization.

In the present disclosure, the olefin (represented by Formula CH₂=CHR, wherein R is hydrogen or a C₁-C₁₂ alkyl) may undergo either homopolymerization or copolymerization of ethylene and an alpha-olefin monomer. The polymerization process may adopt conventional procedures known in the art, and thus detailed descriptions are omitted herein.

Compared with the prior art, the technical solution of the present disclosure achieves at least the following beneficial effects: the catalyst employs a hydrogenated 1,10-phenanthroline derivative as an internal electron donor, and exhibits excellent catalytic activity and hydrogen response. In addition, when the catalyst employing the internal electron donor according to the present disclosure is used for an olefin polymerization, the resultant polypropylene features broader molecular weight distribution, higher isotactic index, and relatively lower xylene-soluble fraction.

### EXAMPLES

For a better understanding of the technical features and merits of the present disclosure, preferred embodiments thereof are described in detail below. Although the preferred embodiments of the present disclosure have been described in the examples, it should be understood that the present disclosure can be implemented in various forms and should not be limited by the embodiments set forth herein.

Unless otherwise specified, all experimental procedures described in the Examples are performed in accordance with conventional procedures well known in the art.

The following starting materials and reagents used therein are all commercially available.

Details for part of the starting materials are listed below:
Internal electron donor C1, DNBP (di-n-butyl phthalate), commercially available;
Internal electron donor C2 (systematic name: phenyl 2-(N-methylbenzamido)benzoate with structure as shown in Table 1 or 2), commercially available from Beijing InnoChem Science & Technology Co., Ltd.;
Internal electron donor C3 (systematic name: 1,2-dibenzoylphenylenediamine with structure as shown in Table 1 or 2), commercially available from Beijing InnoChem Science & Technology Co., Ltd.;
Internal electron donor C4 (systematic name: 1,2-di[(N-tert-butyl)benzoyl]phenylenediamine with structure as shown in Table 1 or 2), commercially available from Beijing InnoChem Science & Technology Co., Ltd.;
Internal electron donors ID1 to ID5 were all laboratory-synthesized with their respective structures shown in Table 1 or 2;
Substituted or unsubstituted 2,3,4,7,8,9-hexahydro-1,10-phenanthroline, benzoyl chloride, o-methylbenzoyl chloride, methyl chloroformate, all purchased from Beijing InnoChem Science & Technology Co., Ltd.

Based on the structure shown in Table 1 or 2, the internal electron donors ID1 to ID5 have the systematic names listed below:
Internal electron donor ID1, CAS No.: 1464216-94-1, 1,10-di(benzoyl)-2,3,4,7,8,9-hexahydro-1,10-phenanthroline;
Internal electron donor ID2, 1,10-bis(o-methylbenzoyl)-2,3,4,7,8,9-hexahydro-1,10-phenanthroline;
Internal electron donor ID3, 1,10-bis(methoxycarbonyl)-2,3,4,7,8,9-hexahydro-1,10-phenanthroline;
Internal electron donor ID4, 1,10-bis(methoxycarbonyl)-5-adamantyl-2,3,4,7,8,9-hexahydro-1,10-phenanthroline;
Internal electron donor ID5, 1,10-di(benzoyl)-5-adamantyl-2,3,4,7,8,9-hexahydro-1,10-phenanthroline.

### Preparation of Internal Electron Donors

### Preparation Example 1

The synthesis procedure for internal electron donor ID1: 18.8 g (100 mmol) of 2,3,4,7,8,9-hexahydro-1,10-phenanthroline was charged into a 250 mL single-necked flask, followed by addition of 100 mL tetrahydrofuran and 40 mL (300 mmol) triethylamine. Under ice-water bath cooling, 23 mL (200 mmol) of benzoyl chloride was slowly added dropwise into the reaction mixture. After completion of the dropwise addition, the mixture was allowed to react at room temperature for 3 hours. After filtration, the resulting mother liquor was concentrated, the resultant solid was washed three times with 50 mL of methanol, and recrystallized from ethyl acetate to finally give 36.4g of ID1 product with a yield of 92%.

### Preparation Example 2

The synthesis procedure for internal electron donor ID2 was identical to that described in Preparation Example 1, except that benzoyl chloride was replaced with o-methylbenzoyl chloride.

### Preparation Example 3

The synthesis procedure for internal electron donor ID3 was identical to that described in Preparation Example 1, except that benzoyl chloride was replaced with methyl chloroformate.

### Preparation Example 4

The synthesis procedure for internal electron donor ID4 was identical to that described in Preparation Example 1, except that 2,3,4,7,8,9-hexahydro-1,10-phenanthroline was replaced with 5-adamantyl-2,3,4,7,8,9-hexahydro-1,10-phenanthroline and benzoyl chloride was replaced with methyl chloroformate.

### Preparation Example 5

The synthesis procedure for internal electron donor ID5 was identical to that described in Preparation Example 1, except that the 2,3,4,7,8,9-hexahydro-1,10-phenanthroline was replaced with 5-adamantyl-2,3,4,7,8,9-hexahydro-1,10-phenanthroline.

### Preparation of Catalysts and Polymers

### Catalyst Preparation Example 1

A custom-made 250 ml 6-necked reactor equipped with a stirrer and a filter unit was thoroughly purged with nitrogen, followed by charging 2.2 g of diethoxymagnesium and 50 mL of chlorobenzene. The system was maintained at -10°C and 100 mL of titanium tetrachloride/chlorobenzene solution (with a volume ratio of 1:1) was added dropwise. After completion of the addition, the mixture was slowly heated to 90°C, then a chlorobenzene solution of 1.5 g of internal electron donor ID1 was injected into the reactor with a syringe. The mixture was heated to 110°C and held for 1 hour. The mother liquor was fully filtered with pressure. Another 100 mL titanium tetrachloride/chlorobenzene solution (with a volume ratio of 1:1) was added dropwise, then the mixture was allowed to react at a constant temperature of 110°C for 1 hour before full pressure filtration again. Finally 100 mL titanium tetrachloride/chlorobenzene solution (with a volume ratio of 1:1) was added dropwise and the mixture was allowed to react at a constant temperature of 110°C for 0.5 hours, followed by complete removal of mother liquor by pressure filtration. The collected solid was washed three times with 100 mL of n-heptane. After removal of n-heptane by filtration, the solid was dried to afford the solid catalyst component 1 as a powder.

The contents of titanium and internal electron donor in the solid catalyst component 1, and the structure of the internal electron donor are shown in Tables 1 and 2 below.

### Olefin Polymerization Example a1

Propylene polymerization was carried out in a 2L stainless steel autoclave in the laboratory.

Step 1, the autoclave was purged with high-purity nitrogen (H₂O <1 ppm, O₂ <1 ppm); then, 0.55 L of liquid propylene and 0.2 g of hydrogen were charged sequentially at room temperature, and the system was subsequently heated to 70°C;

Step 2: prior to the autoclave temperature reaching 70°C, 0.75 ml of 1M triethylaluminum in heptane solution, 1.1 ml of 0.05M D-donor in hexane solution, and 6 mg of the solid catalyst component 1 (i.e., Z-N catalyst containing the internal electron donor ID1) prepared above were pre-complexed for 5 minutes. Once the autoclave temperature reached 70°C, the mixture obtained from the pre-complexation was charged into the autoclave and polymerization timing was initiated;

Step 3, after 60 minutes of reaction, the autoclave was vented and cooled down, and the reaction was quenched; the resulting polypropylene resin was discharged from the autoclave and vacuum dried at 30°C for 2 hours to afford the polypropylene product.

The resultant polypropylene product was characterized to determine its polymer properties, and the test results are shown in Table 2.

### Olefin Polymerization Example b1

Propylene polymerization was carried out in one 2L stainless steel autoclave in the laboratory:
Step 1, the autoclave was first purged with high-purity nitrogen (H₂O <1 ppm, O₂ <1 ppm ); then, 0.55 L of liquid propylene and 1.2 g of hydrogen were charged sequentially at room temperature, and the system was subsequently heated to 70°C;
Step 2: prior to the autoclave temperature reaching 70°C, 0.75 ml of 1M triethylaluminum in heptane solution, 1.1 ml of 0.05M D-donor in hexane solution, and 6 mg of the solid catalyst component 1 (i.e., Z-N catalyst containing the internal electron donor ID1) prepared above were pre-complexed for 5 minutes. Once the autoclave temperature reached 70°C, the mixture obtained from the pre-complexation was charged into the autoclave and polymerization timing was initiated;
Step 3, after 60 minutes of reaction, the autoclave was vented and cooled down, and the reaction was quenched; the polypropylene resin was discharged from the autoclave and vacuum dried at 30°C for 2 hours to afford the polypropylene product.

The resultant polypropylene product was characterized to determine its polymer properties, and the test results are shown in Table 2.

### Catalyst Preparation Example 2

The synthetic procedure for solid catalyst component was identical to that described in Catalyst Preparation Example 1, except that the internal electron donor ID1 was replaced with ID2. All remaining operating steps were kept consistent with Catalyst Preparation Example 1 to afford solid catalyst component 2 (i.e., Z-N catalyst containing internal electron donor ID2).

The contents of titanium and internal electron donor in the solid catalyst component 2, and the structure of the internal electron donor are shown in Tables 1 and 2 below.

### Olefin Polymerization Example a2

Propylene polymerization was carried out in accordance with Polymerization Example a1, except that the solid catalyst component 1 (i.e., Z-N catalyst containing internal electron donor ID1) in the polymerization was replaced with the solid catalyst component 2 (i.e., Z-N catalyst containing internal electron donor ID2).

### Olefin Polymerization Example b2

Propylene polymerization was carried out in accordance with Polymerization Example b1, except that the solid catalyst component 1 (i.e., Z-N catalyst containing internal electron donor ID1) in the polymerization experiment was replaced with the solid catalyst component 2 (i.e., Z-N catalyst containing internal electron donor ID2).

The resultant polypropylene product was characterized to determine its polymer properties, and the test results are shown in Table 2.

### Catalyst Preparation Example 3

The synthetic procedure for solid catalyst component was identical to that described in Catalyst Preparation Example 1, except that the internal electron donor ID1 was replaced with ID3. All remaining operating steps were kept consistent with Catalyst Preparation Example 1 to afford solid catalyst component 3 (i.e., Z-N catalyst containing internal electron donor ID3.

The contents of titanium and internal electron donor in solid catalyst component 3, and the structure of the internal electron donor are shown in Tables 1 and 2 below.

### Olefin Polymerization Example a3

Propylene polymerization was carried out in accordance with Polymerization Example a1, except that the solid catalyst component 1 (i.e., Z-N catalyst containing internal electron donor ID1) in the polymerization experiment was replaced with the solid catalyst component 3 (i.e., Z-N catalyst containing internal electron donor ID3).

### Olefin Polymerization Example b3

Propylene polymerization was carried out in accordance with Polymerization Example b1, except that the solid catalyst component 1 (i.e., Z-N catalyst containing internal electron donor ID1) in the polymerization experiment was replaced with the solid catalyst component 3 (i.e., Z-N catalyst containing internal electron donor ID3).

The obtained polypropylene product was characterized, and the test results are shown in Table 2.

### Catalyst Preparation Example 4

The synthetic procedure for the solid catalyst component was identical to that described in Catalyst Preparation Example 1, except that the internal electron donor ID1 was replaced with ID4. All remaining operating steps were kept consistent with Catalyst Preparation Example 1 to afford solid catalyst component 4 (i.e., Z-N catalyst containing internal electron donor ID4).

The contents of titanium and internal electron donor in solid catalyst component 4, and the structure of the internal electron donor are shown in Tables 1 and 2 below.

### Olefin Polymerization Example a4

Propylene polymerization was carried out in accordance with Polymerization Example a1, except that the solid catalyst component 1 (i.e., Z-N catalyst containing internal electron donor ID1) in the polymerization experiment was replaced with the solid catalyst component 4 (i.e., Z-N catalyst containing internal electron donor ID4).

### Olefin Polymerization Example b4

Propylene polymerization was carried out in accordance with Polymerization Example b1, except that the solid catalyst component 1 (i.e., Z-N catalyst containing internal electron donor ID1) in the polymerization experiment was replaced with the solid catalyst component 4 (i.e., Z-N catalyst containing internal electron donor ID4).

The resultant polypropylene product was characterized to determine its polymer properties, and the test results are shown in Table 2.

### Catalyst Preparation Example 5

The synthetic procedure for the solid catalyst component was identical to that described in Catalyst Preparation Example 1, except that the internal electron donor ID1 was replaced with ID5. All remaining operating steps were kept consistent with Catalyst Preparation Example 1 to afford solid catalyst component 5 (i.e., Z-N catalyst containing internal electron donor ID5).

The contents of titanium and internal electron donor in solid catalyst component 5, and the structure of the internal electron donor are shown in Tables 1 and 2 below.

### Olefin Polymerization Example a5

Propylene polymerization was carried out in accordance with Polymerization Example a1, except that the solid catalyst component 1 (i.e., Z-N catalyst containing internal electron donor ID1) in the polymerization experiment was replaced with the solid catalyst component 5 (i.e., Z-N catalyst containing internal electron donor ID5).

### Olefin Polymerization Example b5

Propylene polymerization was carried out in accordance with Polymerization Example b1, except that the solid catalyst component 1 (i.e., Z-N catalyst containing internal electron donor ID1) in the polymerization experiment was replaced with the solid catalyst component 5 (i.e., Z-N catalyst containing internal electron donor ID5).

The obtained polypropylene product was characterized, and the test results are shown in Table 2.

### Catalyst Preparation Comparative Example 1

The synthetic procedure for the solid catalyst component was identical to that described in Catalyst Preparation Example 1, except that the internal electron donor ID1 was replaced with C1. All remaining operating steps were kept consistent with Catalyst Preparation Example 1 to afford solid catalyst component 1' (i.e., Z-N catalyst containing internal electron donor C1).

The contents of titanium and internal electron donor in solid catalyst component 1', and the structure of the internal electron donor are shown in Tables 1 and 2 below.

### Olefin Polymerization Comparative Example a1'

Propylene polymerization was carried out in accordance with Polymerization Example a1, except that the solid catalyst component 1 (i.e., Z-N catalyst containing internal electron donor ID1) in the polymerization experiment was replaced with the solid catalyst component 1' (i.e., Z-N catalyst containing internal electron donor C1).

### Olefin Polymerization Comparative Example b1'

Propylene polymerization was carried out in accordance with Polymerization Example b1, except that the solid catalyst component 1 (i.e., Z-N catalyst containing internal electron donor ID1) in the polymerization experiment was replaced with the solid catalyst component 1' (i.e., Z-N catalyst containing internal electron donor C1).

The obtained polypropylene product was characterized, and the test results are shown in Table 2.

### Catalyst Preparation Comparative Example 2

The synthetic procedure for the solid catalyst component was identical to that described in Catalyst Preparation Example 1, except that the internal electron donor ID1 was replaced with C2. All remaining operating steps were kept consistent with Catalyst Preparation Example 1 to afford solid catalyst component 2' (i.e., Z-N catalyst containing internal electron donor C2).

The contents of titanium and internal electron donor in solid catalyst component 2', and the structure of the internal electron donor are shown in Tables 1 and 2 below.

### Olefin Polymerization Comparative Example a2'

Propylene polymerization was carried out in accordance with Polymerization Example a1, except that the solid catalyst component 1 (i.e., Z-N catalyst containing internal electron donor ID1) in the polymerization experiment was replaced with the solid catalyst component 2' (i.e., Z-N catalyst containing internal electron donor C2).

### Olefin Polymerization Comparative Example b2'

Propylene polymerization was carried out in accordance with Polymerization Example 1, except that the solid catalyst component 1 (i.e., Z-N catalyst containing internal electron donor ID1) in the polymerization experiment was replaced with the solid catalyst component 2'(i.e., Z-N catalyst containing internal electron donor C2).

The obtained polypropylene product was characterized, and the test results are shown in Table 2.

### Catalyst Preparation Comparative Example 3

The synthetic procedure for the solid catalyst component was identical to that described in Catalyst Preparation Example 1, except that the internal electron donor ID1 was replaced with C3. All remaining operating steps were kept consistent with in Catalyst Preparation Example 1 to afford solid catalyst component 3' (i.e., Z-N catalyst containing internal electron donor C3).

The contents of titanium and internal electron donor in solid catalyst component 3', and the structure of the internal electron donor are shown in Tables 1 and 2 below.

### Olefin Polymerization Comparative Example a3'

Propylene polymerization was carried out in accordance with Polymerization Example a1, except that the solid catalyst component 1 (i.e., Z-N catalyst containing internal electron donor ID1) in the polymerization experiment was replaced with the solid catalyst component 3' (i.e., Z-N catalyst containing internal electron donor C3).

### Olefin Polymerization Comparative Example b3'

Propylene polymerization was carried out in accordance with Polymerization Example b1, except that the solid catalyst component 1 (i.e., Z-N catalyst containing internal electron donor ID1) in the polymerization experiment was replaced with the solid catalyst component 3' (i.e., Z-N catalyst containing internal electron donor C3).

The obtained polypropylene product was characterized, and the test results are shown in Table 2.

### Catalyst Preparation Comparative Example 4

The synthetic procedure for the solid catalyst component was identical to that described in Catalyst Preparation Example 1, except that the internal electron donor ID1 was replaced with C4. All remaining operating steps were kept consistent with in Catalyst Preparation Example 1 to afford solid catalyst component 4' (i.e., Z-N catalyst containing internal electron donor C4).

The contents of titanium and internal electron donor in solid catalyst component 4', and the structure of the internal electron donor are shown in Tables 1 and 2 below.

### Olefin Polymerization Comparative Example a4'

Propylene polymerization was carried out in accordance with Polymerization Example a1, except that the solid catalyst component 1 (i.e., Z-N catalyst containing internal electron donor ID1) in the polymerization experiment was replaced with the solid catalyst component 4' (i.e., Z-N catalyst containing internal electron donor C4).

### Olefin Polymerization Comparative Example b4'

Propylene polymerization was carried out in accordance with Polymerization Example b1, except that the solid catalyst component 1 (i.e., Z-N catalyst containing internal electron donor ID1) in the polymerization experiment was replaced with the solid catalyst component 4' (i.e., Z-N catalyst containing internal electron donor C4).

The obtained polypropylene product was characterized, and the test results are shown in Table 2.

The polymer products from the Examples and Comparative Examples were characterized in accordance with the test procedures specified below:
1. Melt flow rate (MFR): measured according to ASTM D1238;
2. Isotactic index: determined with an automatic xylene-soluble fraction analyzer (CRYSTEX QC) from Polymer Char Company. The value of the isotactic index was calculated by subtracting the measured content of the xylene-soluble fraction from 1;
3. Molecular weight and molecular weight distribution: determined using the High-temperature gel permeation chromatograph (GPC-IR6) from Polymer Char Company;
4. Catalytic activity: As commonly known in this field, catalyst activity is calculated by dividing the mass of collected polymer by the initial catalyst amount or via other conventional calculation routes.

**Table 1**

| Catalyst Examples | Internal electron donor ID | Content of ID (wt%) | Content of Ti (wt%) | Polymerization Examples | Amount of Hydrogen (g) | Activity (Kg/g Cat.h⁻¹) |
|---|---|---|---|---|---|---|
| Example 1 | 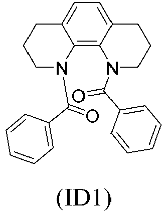 | 9.8 | 3.5 | Example a1 | 0.2g | 47.8 |
| | | | | Example b1 | 1.2g | 43.3 |
| Example 2 | 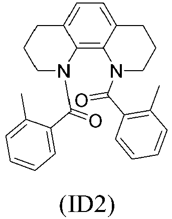 | 8.2 | 3.1 | Example a2 | 0.2g | 45.2 |
| | | | | Example b2 | 1.2g | 41.8 |
| Example 3 | 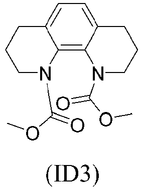 | 7.3 | 3.2 | Example a3 | 0.2g | 39.2 |
| | | | | Example b3 | 1.2g | 38.7 |
| Example 4 | 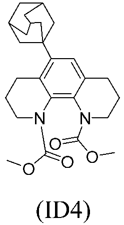 | 8.6 | 3.4 | Example a4 | 0.2g | 37.6 |
| | | | | Example b4 | 1.2g | 38.2 |
| Example 5 | 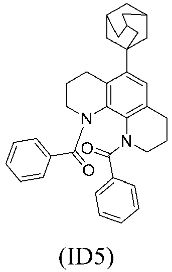 | 9.2 | 3.3 | Example a5 | 0.2g | 46.7 |
| | | | | Example b5 | 1.2g | 46.2 |
| Comparative Example 1 | DNBP (C1) | 13.5 | 2.7 | Comparative Example a1' | 0.2g | 37.1 |
| | | | | Comparative Example b1' | 1.2g | 35.6 |
| Comparative Example 2 | 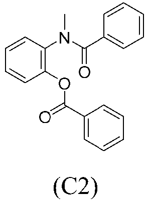 | 9.3 | 2.9 | Comparative Example a2' | 0.2g | 39.5 |
| | | | | Comparative Example b2' | 1.2g | 40.1 |
| Comparative Example 3 | 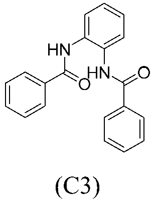 | 10.2 | 3.7 | Comparative Example a3' | 0.2g | 43.5 |
| | | | | Comparative Example b3' | 1.2g | 44.0 |
| Comparative Example 4 | 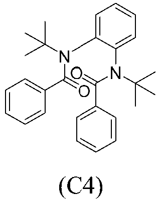 | 11.4 | 3.3 | Comparative Example a4' | 0.2g | 43.1 |
| | | | | Comparative Example b4' | 1.2g | 43.7 |

**Table 2**

| Catalyst Examples | Internal electron donor ID | Polymerization Examples | Isotactic index (I.I.) (wt%) | Melt Flow Rate (MFR) (g/10 min) | Molecular weight distribution |
|---|---|---|---|---|---|
| Example 1 | 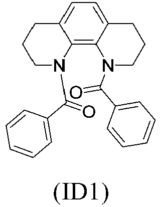 | Example a1 | 98.4 | 5.5 | 6.9 |
| | | Example b1 | 98.1 | 99.2 | 7.0 |
| Example 2 | 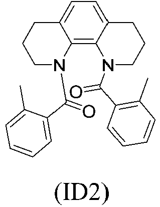 | Example a2 | 98.3 | 5.7 | 6.4 |
| | | Example b2 | 97.9 | 106.3 | 6.6 |
| Example 3 | 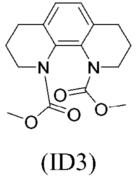 | Example a3 | 98.2 | 5.9 | 7.3 |
| | | Example b3 | 97.8 | 89.7 | 6.9 |
| Example 4 | 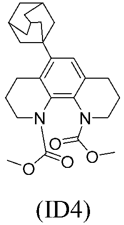 | Example a4 | 98.9 | 3.7 | 6.8 |
| | | Example b4 | 98.0 | 138.9 | 6.9 |
| Example 5 | 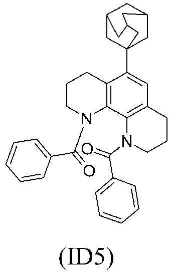 | Example a5 | 98.8 | 4.2 | 6.5 |
| | | Example b5 | 98.2 | 157.1 | 6.9 |
| Comparative Example 1 | DNBP (C1) | Comparative Example a1' | 98.5 | 3.2 | 5.6 |
| | | Comparative Example b1' | 97.8 | 43.96 | 5.2 |
| Comparative Example 2 | 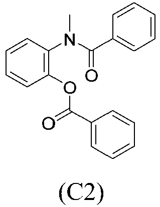 | Comparative Example a2' | 98.1 | 3.7 | 6.3 |
| | | Comparative Example b2' | 97.2 | 67.2 | 6.1 |
| Comparative Example 3 | 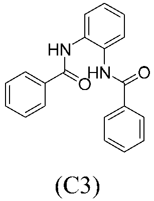 | Comparative Example a3' | 97.4 | 4.8 | 6.3 |
| | | Comparative Example b3' | 96.7 | 54.2 | 6.5 |
| Comparative Example 4 | 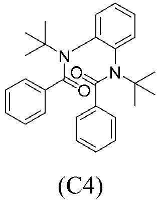 | Comparative Example a4' | 98.1 | 5.1 | 6.7 |
| | | Comparative Example b4' | 97.3 | 68.3 | 6.4 |

The polymerization results in the tables above show that the polymerization catalysts prepared using the hydrogenated 1,10-phenanthroline derivatives as internal electron donors exhibit excellent catalytic activity and hydrogen response when used for propylene polymerization.

Under the same preparation conditions, the catalysts prepared using the hydrogenated 1,10-phenanthroline derivatives as internal electron donors in the present disclosure exhibit superior catalytic activity and hydrogen response relative to catalysts using other noncyclic aromatic amine compounds as internal electron donors, and the polymers obtained by polymerization using the catalyst according to the present disclosure have higher isotactic indexes. In addition, when the catalysts prepared using such internal electron donors in the present disclosure are utilized in olefin polymerization, the molecular weight distribution of the obtained polypropylene is generally broader than that of the polypropylene obtained by applying the catalysts prepared using phthalates as internal electron donors in olefin polymerization.

The foregoing describes the embodiments of the present disclosure, which are illustrative rather than exhaustive and shall not be construed as limiting the disclosed examples. Various modifications and variations will be readily apparent to those skilled in the art without departing from the spirit and scope of the disclosure.

## Claims

1. An internal electron donor for an olefin polymerization catalyst, wherein the internal electron donor is at least one selected from the compounds represented by Formula I below: 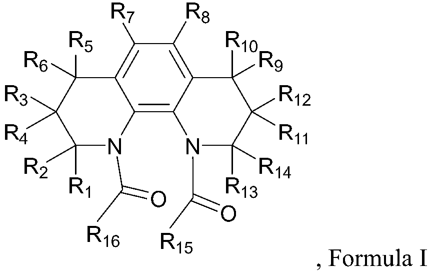 in Formula I:
R₁ to R₁₄ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl; two or more of R₁ to R₁₄ may be linked together to form cyclic structure(s) or unsaturated bond(s);
R₁₅ and R₁₆ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl, and benzyl, wherein the heteroatom is selected from N, O, S, P and Si; and heteroaryl;
any hydrogen atom on benzene ring of aryl, arylalkyl or alkylaryl may each be optionally substituted with halogen.

2. The internal electron donor according to claim 1, wherein the internal electron donor is at least one selected from the compounds represented by Formula (II) below: 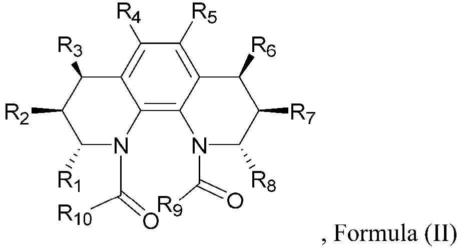 in Formula (II):
R₁ to R₈ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl; two or more of R₁ to R₈ may be linked together to form cyclic structure(s) or unsaturated bond(s);
R₉ and R₁₀ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl.

3. The internal electron donor according to claim 2, wherein in Formula (II), each of R₉ and R₁₀ is independently selected from phenyl, butyl and ethoxy.

4. A solid catalyst component for olefin polymerization, comprising a titanium compound, a magnesium compound, and at least one of the internal electron donors of any one of claims 1 to 3.

5. The solid catalyst component according to claim 4, wherein a method for preparing the solid catalyst component comprises: bringing a magnesium compound precursor, the titanium compound and at least one of the internal electron donors into contact and reaction to obtain the solid catalyst component; wherein,
the magnesium compound precursor is at least one selected from the compounds represented by Formula XₙMg(OR)₂₋ₙ, the compounds represented by Formula MgCl₂·mROH, MgCl₂/SiO₂ mixtures, MgCl₂/Al₂O₃ mixtures, and mixtures of magnesium halides and titanium alkoxides, wherein m ranges from 0.1 to 6, n is no less than 0 and no more than 2, X is halogen, R is hydrogen or a C₁-C₈ hydrocarbyl group;
the titanium compound is represented by Formula TiXₙ(OR)₄₋ₙ, wherein R is selected from C₁-C₂₀ hydrocarbyl, X is halogen, and n ranges from 1 to 4.

6. A polymerization catalyst for olefin polymerization, wherein the polymerization catalyst comprises a product formed by reacting the following starting materials:
(a) at least one of the solid catalyst components of claim 4 or 5;
(b) at least one of the organo-aluminum compounds represented by Formula AlRₙX₍₃₋ₙ₎, wherein R is selected from hydrogen and C₁-C₂₀ hydrocarbyl group; X is selected from halogen, and n is an integer of no less than 0 and no more than 3;
(c) at least one of the alkoxysilane compounds represented by Formula RₙSi(OR¹)₄₋ₙ, wherein R and R¹ are the same or different, and each is independently selected from C₁-C₁₈ hydrocarbyl group and halogenated hydrocarbyl group, and C₁-C₁₀ substituent optionally containing heteroatom; and n is an integer satisfying 0≦n≦3.

7. The polymerization catalyst according to claim 6, wherein in the organo-aluminum compounds represented by Formula AlRₙX₍₃₋ₙ₎, R is selected from hydrogen and C₁-C₁₂ hydrocarbyl group.

8. The polymerization catalyst according to claim 6 or 7, wherein the organo-aluminum compound is selected from trialkylaluminum compounds, preferably one or more selected from trimethylaluminum, triethylaluminum, triisobutylaluminum, tri-n-butylaluminum, tri-n-hexylaluminum, and trioctylaluminum.

9. The polymerization catalyst according to any one of claims 6 to 8, wherein in the alkoxysilane compound represented by Formula RₙSi(OR¹)₄₋ₙ, each of R and R¹ is independently selected from C₁-C₁₈ alkyl, C₃-C₁₈ cycloalkyl, C₆-C₁₈ aryl, and C₁-C₁₈ haloalkyl.

10. Use of the polymerization catalyst of any one of claims 6 to 9 in olefin polymerization.
